# EUROPEAN PATENT APPLICATION

(11) **EP 4 767 930 A1**
(43) Date of publication of application: **01.07.2026**
(21) Application number: 25223861.3
(22) Date of filing: 16.12.2025
(51) Int. Cl.: A61B 5/055, A61B 5/00, A61B 6/46, G01R 33/28

(54) **MEDICAL IMAGING APPARATUS, MEDICAL IMAGING SUPPORT METHOD, AND PROGRAM**

(30) Priority: 26.12.2024 JP 2024230905
(71) Applicant: FUJIFILM Corporation, Tokyo 106-8620 (JP)
(72) Inventor: IKEGAWA, Ayaka, Tokyo, 1068620 (JP)
(74) Representative: Dehns Germany Partnerschaft mbB

(57) **Abstract**

Provided are a medical imaging apparatus, a medical imaging support method, and a program that can notify a user of a plurality of notifications from a subject. A medical imaging apparatus according to the present disclosure captures a medical image of a subject, identifies a first motion performed by the subject inserted into a bore of a gantry based on information acquired by a motion sensor, compares the identified first motion with a plurality of second motions registered in a memory, and notifies the user of notification content corresponding to the second motion that matches the identified first motion among the plurality of second motions registered in the memory.

## Description

### BACKGROUND OF THE INVENTION

### 1. Field of the Invention

The present invention relates to a medical imaging apparatus, a medical imaging support method, and a program, and particularly to a technique of detecting a motion of a subject.

### 2. Description of the Related Art

As a device that images and displays an internal tissue of a subject such as a human body, a magnetic resonance imaging (MRI) apparatus is known.

JP2024-082179A discloses an MRI apparatus that detects a movement of a subject and calculates a body movement by using a video from a camera installed in an examination space of the MRI apparatus.

JP2015-112152A discloses: a body movement sensor that is mounted on a subject, senses a body movement of the subject, and generates a sensing signal; and a body movement determination device that determines whether or not the body movement sensed by the body movement sensor is a predetermined body movement by comparing the predetermined standard pattern with the sensing signal generated by the body movement sensor, and generates a body movement detection signal in a case where it is determined that the body movement is the predetermined body movement.

### SUMMARY OF THE INVENTION

During the MRI examination, the subject grips a buzzer called an emergency call button to sound a buzzer and notify an operator in an operation room. The subject can sound the buzzer sound in a case where the subject wants to complain about a deterioration in mood or a physical discomfort, but the buzzer can only represent a state as on or off (presence or absence of notification). In addition, there is a subject who has difficulty gripping the buzzer.

In addition, in the MRI apparatus disclosed in JP2024-082179A and the body movement determination device disclosed in JP2015-112152A, the body movement of the subject is detected, but the subject cannot communicate the intention.

The present invention has been made in view of such circumstances, and an object of the present invention is to provide a medical imaging apparatus, a medical imaging support method, and a program that can notify a user of a plurality of notifications from a subject.

In order to achieve the above object, according to a first aspect of the present disclosure, there is provided a medical imaging apparatus that captures a medical image of a subject, the medical imaging apparatus comprising: a gantry having a bore into which the subject placed on a patient table is inserted; a motion sensor that acquires information used to identify a first motion performed by the subject; a memory in which a plurality of second motions and notification content corresponding to each of the plurality of second motions are registered; and a processor, in which the processor is configured to: identify the first motion performed by the subject inserted into the bore based on the information acquired by the motion sensor; compare the identified first motion with the plurality of second motions registered in the memory; and notify the user of the notification content corresponding to the second motion that matches the identified first motion among the plurality of second motions registered in the memory.

According to a second aspect of the present disclosure, in the medical imaging apparatus according to the first aspect, it is preferable that the plurality of second motions include the same type of motions having different magnitudes.

According to a third aspect of the present disclosure, in the medical imaging apparatus according to the first or second aspect, it is preferable that the plurality of second motions include the same type of motions having different durations.

According to a fourth aspect of the present disclosure, in the medical imaging apparatus according to any one of the first to third aspects, it is preferable that the motion sensor includes any one of an optical camera that images the subject, a sensor disposed on the patient table, or a sensor attached to a body of the subject.

According to a fifth aspect of the present disclosure, in the medical imaging apparatus according to the fourth aspect, it is preferable that the sensor disposed on the patient table includes a touch sensor or a pressure sensor.

According to a sixth aspect of the present disclosure, in the medical imaging apparatus according to the fourth aspect, it is preferable that the sensor attached to the body of the subject includes at least one of an acceleration sensor, a gyro sensor, or a pressure sensor.

According to a seventh aspect of the present disclosure, in the medical imaging apparatus according to any one of the first to sixth aspects, it is preferable that the processor is configured to combine information acquired by at least one of site specification using a skeleton extraction technique, motion tracking, or pattern recognition by a trained model with the information acquired by the motion sensor to identify the first motion.

According to an eighth aspect of the present disclosure, in the medical imaging apparatus according to any one of the first to seventh aspects, it is preferable that the processor is configured to: acquire vital information of the subject; and notify the user of predetermined notification content in accordance with the acquired vital information.

According to a ninth aspect of the present disclosure, in the medical imaging apparatus according to any one of the first to eighth aspects, it is preferable that the processor is configured to display the notification content in a pop-up manner on a display device.

According to a tenth aspect of the present disclosure, in the medical imaging apparatus according to any one of the first to ninth aspects, it is preferable that the medical imaging apparatus further comprises: a static magnetic field generation device that generates a static magnetic field; a transmission device that irradiates the subject placed in a static magnetic field space with a high-frequency magnetic field pulse; and a gradient magnetic field generation device that generates a gradient magnetic field in the static magnetic field space.

In order to achieve the above object, according to an eleventh aspect of the present disclosure, there is provided a medical imaging support method of supporting capturing of a medical image of a subject by a medical imaging apparatus including a gantry having a bore into which the subject placed on a patient table is inserted, a motion sensor that acquires information used to identify a first motion performed by the subject, a memory in which a plurality of second motions and notification content corresponding to each of the plurality of second motions are registered, and a processor, the medical imaging support method comprising: causing the processor to execute: identifying the first motion performed by the subject inserted into the bore based on the information acquired by the motion sensor; comparing the identified first motion with the plurality of second motions registered in the memory; and notifying the user of the notification content corresponding to the second motion that matches the identified first motion among the plurality of second motions registered in the memory.

In order to achieve the above object, according to a twelfth aspect of the present disclosure, there is provided a program for causing a computer to execute the medical imaging support method according to the eleventh aspect. The present disclosure also includes a non-transitory computer-readable storage medium in which the program according to the twelfth aspect is stored.

In the medical imaging support method according to the eleventh aspect and the program according to the twelfth aspect, the same specific aspects as the medical imaging apparatus described above can be adopted.

According to an aspect of the present invention, a plurality of notifications can be made from the subject to the user.

### BRIEF DESCRIPTION OF THE DRAWINGS

FIG. 1 is a perspective view showing an external appearance of an MRI apparatus.
FIG. 2 is a diagram showing a schematic configuration of an inside of the MRI apparatus.
FIG. 3 is a diagram showing another example of a disposition of a camera.
FIG. 4 is a block diagram showing an example of a hardware configuration of the information processing apparatus.
FIG. 5 is a diagram showing an example of registration contents of a database.
FIG. 6 is a diagram showing an example of a motion of the subject.
FIG. 7 is a flowchart showing a medical imaging support method according to a first embodiment.
FIG. 8 is a diagram showing an example of a display of a display device.
FIG. 9 is a diagram showing an example of registration contents of a database according to a second embodiment.
FIG. 10 is a flowchart showing a medical imaging support method according to a second embodiment.
FIG. 11 is a block diagram showing a functional configuration of the information processing apparatus.

### DESCRIPTION OF THE PREFERRED EMBODIMENTS

Preferred embodiments of the present invention will be described below in detail with reference to the accompanying drawings. In the present specification, identical reference numerals are denoted by identical components, and duplicate descriptions will be omitted as appropriate.

### Overview of MRI Apparatus

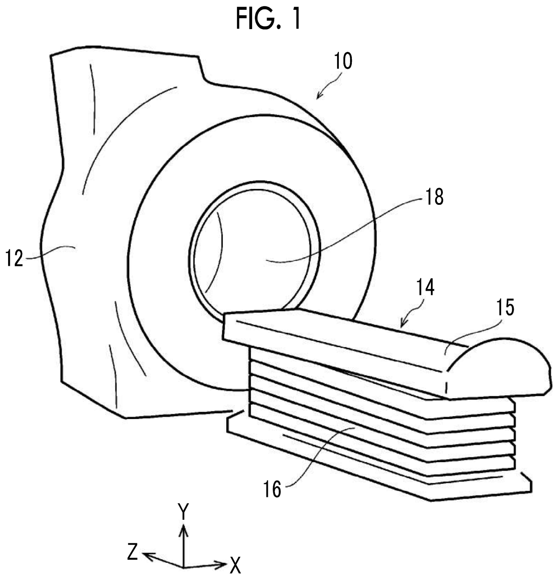

FIG. 1 is a perspective view showing an external appearance of an MRI apparatus 10 that is an example of a medical imaging apparatus. The MRI apparatus 10 comprises a gantry 12 that is an imaging apparatus main body that captures an MRI image that is an example of a medical image, and a patient table 14. The gantry 12 includes a cylindrical imaging space 18, which is called a bore. A static magnetic field generating magnet, a gradient magnetic field coil, and various other coils for generating a magnetic field in the imaging space 18 are disposed in the gantry 12. The static magnetic field generating magnet is an example of a static magnetic field generation device that generates a static magnetic field. The gradient magnetic field coil is an example of a gradient magnetic field generation device that generates a gradient magnetic field in the static magnetic field space.

The patient table 14 comprises a top plate 15 and a leg portion 16 that supports the top plate 15, and is disposed on a front side of the gantry 12. The top plate 15 can be advanced into the imaging space 18 and retracted from the imaging space 18 by a drive mechanism (not shown) provided in the leg portion 16. The patient table 14 may be fixed to the gantry 12 or may be a movable dockable patient table that is attachable to and detachable from the gantry 12.

In the MRI apparatus 10, three axes of the apparatus, which are three-dimensional orthogonal coordinate axes in a real space including the imaging space 18, are defined. A direction of each axis of the three axes of the apparatus is uniquely determined from the gantry 12 and the patient table 14. In FIG. 1, a Z direction is a static magnetic field direction, a Y direction is a vertical direction, and an X direction is a direction orthogonal to the Y direction and the Z direction.

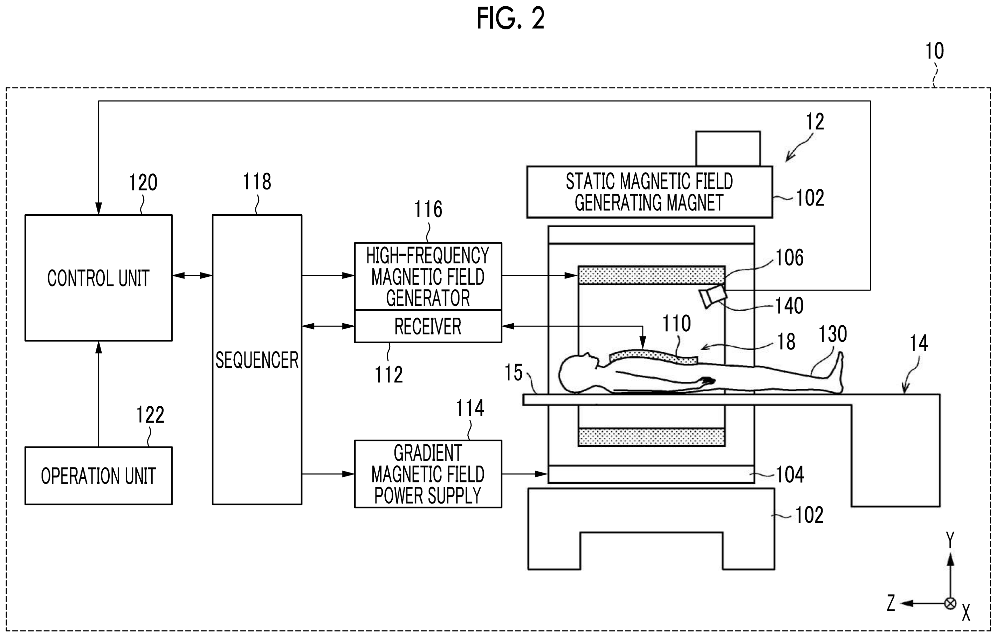

FIG. 2 is a diagram showing a schematic configuration of an inside of the MRI apparatus 10. The MRI apparatus 10 comprises a static magnetic field generating magnet 102, a gradient magnetic field coil 104, a radio frequency (RF) transmit coil 106, a receive coil 110, a receiver 112, a gradient magnetic field power supply 114, a high-frequency magnetic field generator 116, a sequencer 118, a control unit 120, and an operation unit 122. In addition, the MRI apparatus 10 comprises a camera 140 for detecting a motion of a subject 130.

The static magnetic field generating magnet 102 generates a uniform static magnetic field in the imaging space 18. The static magnetic field generating magnet 102 includes a static magnetic field generating source of a permanent magnet type, a normal conducting type, or a superconducting type. The gradient magnetic field coil 104 generates a gradient magnetic field in the imaging space 18. The gradient magnetic field coil 104 is composed of gradient magnetic field coils in three-axis directions of X, Y, and Z, which correspond to a real space coordinate system (stationary coordinate system). Each of the gradient magnetic field coils is connected to a gradient magnetic field power supply 114 and is supplied with a current. As a result, gradient magnetic fields are generated in the three-axis directions of X, Y, and Z.

The subject 130 is placed on the top plate 15 of the patient table 14, and the receive coil 110 corresponding to an examination site is worn. By inserting the top plate 15 with the subject 130 placed thereon into the imaging space 18, the examination site of the subject 130 is positioned at a static magnetic field center in the imaging space 18.

The camera 140 is an example of a motion sensor that detects the motion of the subject 130. The camera 140 is an optical camera that optically captures continuous images at a frame rate equal to or higher than a predetermined frame rate. For example, it is desirable that the camera 140 can capture a video consisting of 30 or more images per second. The camera 140 may be configured to capture a still image.

The camera 140 typically includes an imaging optical system that includes a lens, and an imaging element that captures an optical image formed by the imaging optical system and that converts the optical image into an electrical signal. The imaging element is configured, for example, by using a complementary metal-oxide semiconductor (CMOS) type color image sensor. The imaging element is not limited to the CMOS type and may be a charge-coupled device (CCD) type image sensor. Additionally, the camera 140 may include an image processing circuit that processes the electrical signal obtained from the imaging element to form a digital image. The subject 130 on the patient table 14 is imaged by the camera 140, and the motion of the subject 130 is identified based on the image acquired via the camera 140. In the present specification, the image acquired via the camera 140 is referred to as a "camera image". An infrared camera, a millimeter-wave camera, or the like may be used instead of or in combination with the camera 140.

The RF transmit coil 106 is a coil that irradiates the subject 130 with an RF pulse that is a high-frequency magnetic field pulse, and is an example of a transmission device. The RF transmit coil 106 is connected to the high-frequency magnetic field generator 116 and is supplied with a high-frequency pulse current from the high-frequency magnetic field generator 116.

The sequencer 118 sends commands to the high-frequency magnetic field generator 116 and the gradient magnetic field power supply 114, in accordance with an imaging pulse sequence. As a result, signals, each amplified appropriately, are sent to the RF transmit coil 106 and the gradient magnetic field coil 104.

The high-frequency magnetic field generator 116 is driven in accordance with the command from the sequencer 118, modulates the amplitude of a high-frequency pulse, and supplies the amplified high-frequency pulse current to the RF transmit coil 106. The RF transmit coil 106 applies a pulsed high-frequency magnetic field (RF pulse) to the subject 130 in accordance with the signal from the high-frequency magnetic field generator 116. Preferably, the sequencer 118 sends the command to the receive coil 110 in accordance with the imaging pulse sequence and turns off the receive coil 110 during a period when the RF pulse is applied.

By the application of the high-frequency magnetic field, nuclear magnetic resonance (NMR) phenomena are induced in spins of atoms constituting biological tissues of the subject 130. The receive coil 110 is a multi-channel RF coil unit that includes a plurality of coil elements for receiving nuclear magnetic resonance signals (NMR signals) generated from the subject 130. In FIG. 2, an example of a blanket-type receive coil 110 that is applied to imaging of a chest and an abdomen is shown, but a form of the receive coil 110 may be applied differently depending on the examination site. For example, receive coils for imaging various sites, such as a head, a spine, an abdomen, a leg, and an arm, can be used.

The NMR signal generated from the subject 130 is detected by the receive coil 110, amplified by a preamplifier (not shown) in the receive coil 110, and transmitted to the receiver 112. In the receiver 112, the amplified NMR signal is subjected to analog-to-digital (AD) conversion and necessary signal processing, thereby generating data. The generated data is transmitted to the control unit 120. This data is also referred to as a received signal or measurement data.

A reception-side cable (not shown) through which the NMR signal received by the receive coil 110 is output is connected to the receive coil 110. Although not shown in FIG. 2, a reception-side connector is connected to an end portion of the reception-side cable. The reception-side connector is connected to a patient table-side connector provided on the patient table 14. The patient table-side connector is connected to a patient table-side cable disposed inside the patient table 14, and the patient table-side cable is connected to the control unit 120. As a result, the receive coil 110, the control unit 120, and the sequencer 118 are communicably connected to each other.

The connection between the receive coil 110, and the control unit 120 and the sequencer 118 is not limited to a wired connection, such as via a cable, and can also be established via a wireless connection. In this case, the receive coil 110 or the patient table 14 further includes at least an AD conversion module and a wireless communication module.

The sequencer 118 controls each unit to operate at a pre-programmed timing and intensity. Among programs, a program that particularly describes the timing and the intensity of the RF pulses, the gradient magnetic field, and signal reception is called a pulse sequence. Various pulse sequences are known depending on the purpose, but detailed descriptions thereof will be omitted here.

The control unit 120 accepts various instruction inputs from the operation unit 122 and integrally controls each unit of the MRI apparatus 10 via the sequencer 118, and executes the MRI imaging.

That is, the control unit 120 performs processing such as inverse Fourier transforming the received signal in a spatial frequency domain received from the receiver 112 to convert the received signal into an image in the real space, thereby generating an MRI image.

In addition, the control unit 120 causes the camera 140 to capture an image together with the MRI imaging and acquires the camera image. The control unit 120 detects the motion of the subject 130 based on the camera image and notifies the user of predetermined notification content.

The operation unit 122 includes an input device, such as a mouse and a keyboard, and a display device, such as a liquid crystal display. The operation unit 122 functions as a user interface (UI) for the user such as a technician to launch, stop, and pause the MRI apparatus 10, select a pulse sequence, input imaging conditions and processing conditions, and the like.

The sequencer 118 and the control unit 120 can be configured by using a computer. Additionally, processing functions of the sequencer 118 and the control unit 120 may be implemented by a computer system including a plurality of computers.

### Camera

The camera 140 shown in FIG. 2 is disposed at a position where the camera 140 can image the subject 130 inserted into the imaging space 18. For example, the camera 140 is disposed at a position where the camera 140 captures the subject 130 obliquely from above the subject 130 in the bore, thereby obtaining a video of a relatively wide range including the chest of the subject 130. It is desirable that the camera 140 is configured to image the entire body of the subject 130. It is desirable that a wide-angle lens or a fisheye lens is applied to the imaging optical system of the camera 140.

The imaging space 18 of the gantry 12 has a structure that makes it difficult for light such as indoor illumination light of the shielded room to enter. Therefore, in a case where the camera 140 that images the inside of the bore is used, it is desirable to dispose an illumination device that irradiates the imaging space 18 with light in order to improve a signal-to-noise ratio (SNR) of the image of the camera 140.

In addition, the camera 140 has a structure that cuts electromagnetic noise such that the gantry 12 does not generate noise due to the electromagnetic noise generated by the camera 140. The structure that cuts the electromagnetic noise may be, for example, a shield box.

Further, the camera 140 has a structure that cuts the static magnetic field generated by the static magnetic field generating magnet 102, the rapidly changing gradient magnetic field applied by the gradient magnetic field coil 104, and the high-frequency magnetic field pulse (RF pulse) emitted from the RF transmit coil 106, so as to function as the camera 140 even in a case where these magnetic fields and high-frequency waves are received. The structure that cuts the magnetic field and high-frequency wave may be, for example, a shield box. The camera 140 may preferably be disposed to avoid an irradiation region of the RF pulse.

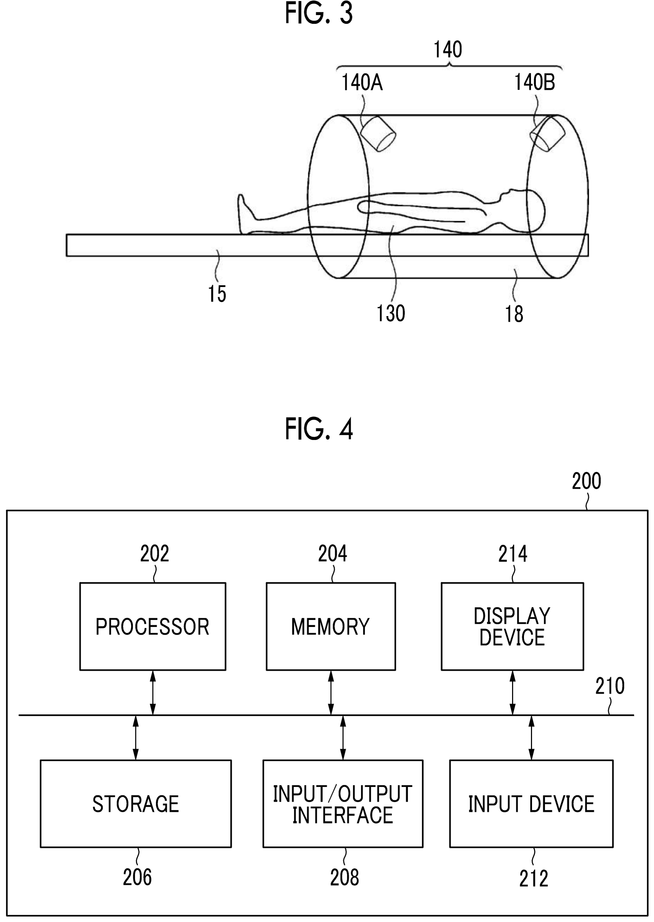

In FIG. 2, an example is shown in which the camera 140 is attached to an entrance side in a case where the subject 130 is inserted into the bore in the gantry 12, but the disposition location and the number of the cameras 140 are not limited to the example of FIG. 2. FIG. 3 is a diagram showing another example of a disposition of a camera 140. As shown in Fig. 3, the camera 140 may be configured such that cameras 140A and 140B are disposed on the entrance side and a far side of the bore, respectively, or may be configured such that only the camera 140B is disposed on the far side of the bore. By imaging the subject 130 from a plurality of directions using a plurality of cameras, it is possible to accurately identify the motion of the subject 130.

The camera 140 may be disposed outside the bore to image the inside of the bore. In addition, instead of or in combination with the camera 140 that image the inside of the bore, a camera may be disposed at a position where the camera can image the outside of the bore. For example, a configuration may be employed in which a camera is disposed on an upper part of a front surface (a surface on a patient table side) of the gantry 12, and the camera images the subject 130 before the subject 130 is moved into the bore or while the subject 130 is being moved into the bore. Additionally, the camera may be attached to a ceiling, a wall, or the like of the shielded room.

### Example of Hardware Configuration of Information Processing Apparatus

FIG. 4 is a block diagram showing an example of a hardware configuration of an information processing apparatus 200 used in the MRI apparatus 10. The information processing apparatus 200 may be a personal computer, a workstation, or a server computer. The computer may be a virtual machine. The information processing apparatus 200 is applied to a console of the MRI apparatus 10 and can function as the control unit 120 and the operation unit 122 (refer to FIG. 2). The information processing apparatus 200 may function as the sequencer 118.

The information processing apparatus 200 comprises a processor 202, a memory 204 that is a main storage device, a storage 206 that is an auxiliary storage device, an input/output interface 208, and a bus 210.

The processor 202 includes a central processing unit (CPU). The processor 202 may include a graphics processing unit (GPU). In addition, the processor 202 may include one or a plurality of pieces of hardware of a digital signal processor (DSP), an application-specific integrated circuit (ASIC), a programmable logic device (PLD), and the like.

The processor 202 is connected to the memory 204, the storage 206, the input/output interface 208, an input device 212, and a display device 214, via the bus 210.

The memory 204 includes a random access memory (RAM). The memory 204 may include a read-only memory (ROM). The storage 206 may be, for example, a hard disk drive (HDD), a solid-state drive (SSD), or a combination of a plurality of these. In addition, the storage 206 may include an external storage device such as a removable medium.

The storage device including the memory 204 and the storage 206 stores programs, data, and the like for implementing various functions of the MRI apparatus 10. The processor 202 executes the programs stored in the memory 204 to implement various functions. The processor 202 integrally controls each unit of the information processing apparatus 200, various devices, units, and the like provided in the MRI apparatus 10, and performs various kinds of processing.

The input/output interface 208 includes a connection interface that can be connected to telecommunication lines such as a local area network, a connection interface that can be connected to external devices, and the like. As the connection interface that can be connected to the external devices, for example, a Universal Serial Bus, a High-Definition Multimedia Interface (HDMI) (HDMI is a registered trademark), and the like can be applied.

The processor 202 communicates with various devices of the MRI apparatus 10 via the input/output interface 208, thereby transmitting and receiving necessary information.

The input device 212 includes, for example, a keyboard, a pointing device such as a mouse, a ten-keypad, various switch buttons, and the like. The input device 212 may include a voice input device. Additionally, the input device 212 may be a touch panel-type input device that is configured integrally with a display screen of the display device 214.

The display device 214 is configured, for example, by using a liquid crystal display, an organic electro-luminescence (OEL) display, a projector, or an appropriate combination thereof. In addition to the MRI image captured by the MRI apparatus 10, various types of information such as the camera image and the coil information are displayed on the display device 214. The display device 214 is used as a part of the user interface in a case where an input from the input device 212 is accepted. The display device 214 is not limited to a single display device and can also be in a multi-display form comprising a plurality of display devices.

### Overview of Procedure of MRI Imaging

A typical imaging procedure by the MRI apparatus 10 is generally as follows.

[Procedure 1] The user such as a technician uses the input device 212 such as a mouse and a keyboard to register information necessary for the examination, such as the subject information, the imaging site, the body position of the subject 130, and the imaging protocol, on a subject registration screen. The term "registration" includes the concept of "setting". The term "user" includes the concepts of terms such as a technician, an operator, and a manipulator.

The subject information includes the name of the subject, a subject code, and the like. The body position of the subject 130 includes an insertion direction of the subject 130 into the gantry 12 and a posture of the subject 130. The imaging protocol includes an imaging type, an order, and the like. A part or all of the information necessary for these examinations may be manually input by the operator via the input device 212 or may be read from information stored in advance in a recording medium or the like. The operator finally confirms automatically set or input parameters and manually sets the parameters by using the input device 212 as necessary.

[Procedure 2] The subject 130 is placed on the top plate 15 of the patient table 14, and the receive coil 110 is worn by the subject 130. The user connects the reception-side connector of the reception-side cable connected to the receive coil 110 to the patient table-side connector on the top plate 15. The top plate 15 is provided with a plurality of patient table-side connectors, and the reception-side connector is connected to the closest patient table-side connector according to the type of the receive coil 110 to be used and the body position of the subject 130. Depending on the combination of the type of the receive coil 110 and the body position of the subject 130, the position of the patient table-side connector to which the reception-side connector can be connected is restricted.

[Procedure 3] After the receive coil 110 is connected to the patient table-side connector, the top plate 15 is moved to the imaging space 18 by using a gantry operation panel, a foot switch, or the like (not shown), and an examination target site of the subject 130 is moved to a static magnetic field center position of the gantry 12.

[Procedure 4] After that, the user presses a start button for issuing an instruction to start imaging, scanogram imaging for acquiring a positioning image is executed. In the scanogram imaging, a plurality of slices are imaged for one or more cross-sections of an axial cross-section, a coronal cross-section, and a sagittal cross-section. A slice thickness in the scanogram imaging may be set to a value greater than a slice thickness in the main imaging. In a case where the imaging start instruction is accepted, an examination window screen is displayed on the display device 214 of the operation unit 122.

[Procedure 5] After the end of the scanogram imaging, the image for positioning is reconstructed, and the image is displayed on the display device 214.

[Procedure 6] The user sets an imaging position of the main imaging based on a scanogram image. In the MRI apparatus 10, an imaging position including a slice position (cross-section position) to be measured in the main imaging is automatically calculated from the scanogram image, and a recommended imaging position is presented to the user. The user confirms the presented slice position and the like and manually adjusts the imaging position by using the input device 212 of the operation unit 122 as necessary. In addition, the user sets imaging parameters to be applied to the main imaging. A part or all of the imaging parameters may be input by the user via the operation unit 122 or may be automatically set or input. The operator finally confirms the automatically set (automatically input) parameters and manually sets the parameters by using the input device 212 of the operation unit 122 as necessary.

[Procedure 7] The main imaging is executed in accordance with the imaging parameters and the imaging position set in this manner.

[Procedure 8] After the main imaging, the image is reconstructed, and the image is displayed on the display device 214.

[Procedure 9] The user sets a window value to a value suitable for diagnosis for the displayed reconstructed image by using the input device 212 to obtain an image to be used for diagnosis.

[Procedure 10] After the imaging is completed, the top plate 15 is retracted from the imaging space 18, and the subject 130 is removed from the gantry 12. Then, the subject 130 is taken off the patient table 14, and the MRI examination ends.

### Medical Imaging Support Method: First Embodiment

The medical imaging support method according to the present disclosure is a method of notifying the user from the subject by performing a motion in accordance with content that the subject of the MRI apparatus 10 wants to notify to the user on the operation room side.

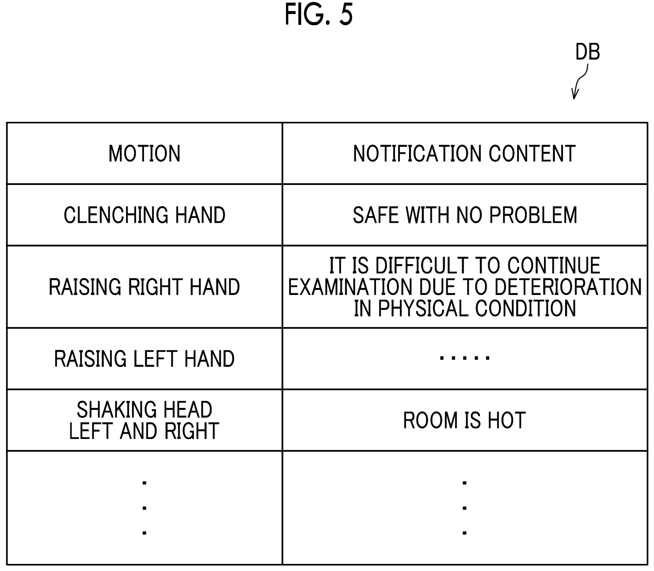

FIG. 5 is a diagram showing an example of registration contents of a database DB used in the medical imaging support method. The database DB is stored in the memory 204 or the storage 206. In the database DB, a plurality of motions (an example of the "plurality of second motions") and notification contents to be notified to the user by the motion are registered in association with each other. The term "motion" refers to an act of moving at least a part of the body of the subject in order to communicate the intention or the emotion of the subject to the user, and is also referred to as a motion pattern, a signal, or a gesture. The term "motion" may include a motion of the subject that is not accompanied by the intention, such as coughing, sneezing, swallowing, and vomiting. The notification content may be a physical discomfort.

In the database DB shown in FIG. 5, "clenching a hand", "raising a right hand", "raising a left hand", "shaking a head left and right", ... are registered as the motions. In addition, in the database DB shown in FIG. 5, "safe with no problem", "It is difficult to continue the examination due to deterioration in physical condition", "......", and "The room is hot" are registered as the notification contents corresponding to "clenching a hand", "raising a right hand", "raising a left hand", and "shaking a head left and right", respectively.

The pair of the motion and the notification content registered in the database DB may be a pair manually registered in advance by the user or the like as one example. In addition, as another example, the pair of the motion and the notification content may be a pair of the past motion of the subject and the notification content corresponding to a tendency indicated by the past motion, and may be a pair automatically extracted and registered by the system.

For example, the information processing apparatus 200 may automatically register the motion that is generally performed by the subject as a sign in the examination of the MRI in the database DB by extracting the motion from an empirical rule of the past motion of the subject in the bore. As a result, the information processing apparatus 200 can register, as the motion, "the number of swallowing per unit time is increased" and, as the corresponding notification content, "there is a tendency of physical discomfort".

As still another example, the database DB may be a trained model that has been trained by machine learning (for example, deep learning). In this case, the trained model is trained by inputting and outputting the pair of the past motion of the subject and the notification content corresponding to the tendency indicated by the past motion. As a result, in a case where the motion of the new subject 130 is input, the trained model outputs the notification content.

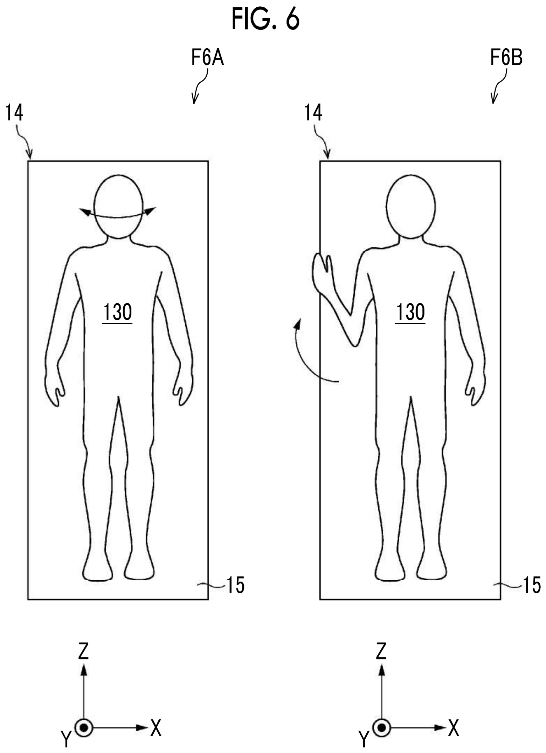

FIG. 6 is a diagram showing an example of a motion of the subject 130. F6A in FIG. 6 shows a state in which the subject 130 is performing the motion of "shaking a head left and right". The subject 130 communicates the intention of "the room is hot" to the user by shaking the head left and right as in F6A.

In addition, F6B in FIG. 6 shows a state in which the subject 130 is performing the motion of "raising a right hand". The subject 130 communicates the intention of "it is difficult to continue the examination due to deterioration in physical condition" to the user by raising the right hand as in F6B.

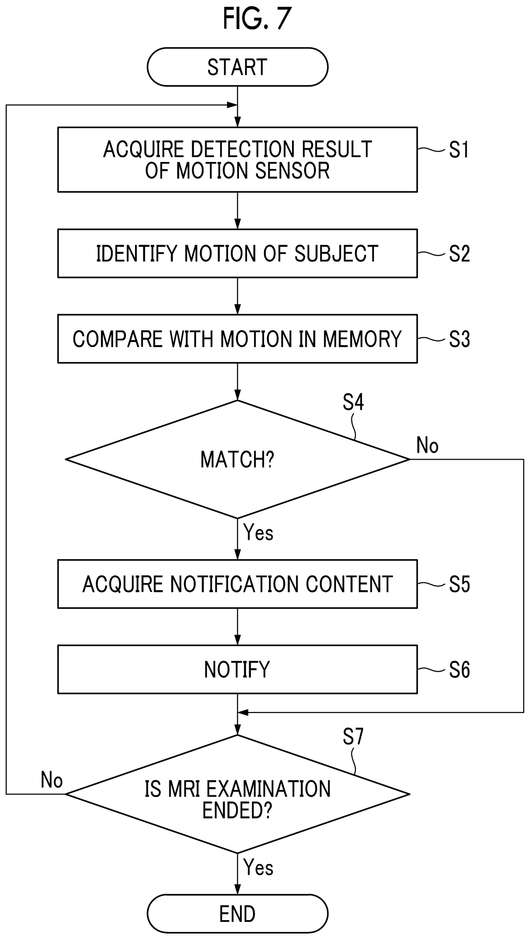

FIG. 7 is a flowchart showing a medical imaging support method according to a first embodiment. The medical imaging support method is realized by the processor 202 executing a medical imaging support program stored in the memory 204. The processor 202 may read out and execute the medical imaging support program stored in a non-transitory and computer-readable storage medium.

The medical imaging support method is mainly performed while the subject 130 is inserted into the bore. The medical imaging support method may be performed while the subject 130 is placed on the top plate 15 of the patient table 14. Here, it is assumed that the processing of the present flowchart is started after the subject 130 is placed on the top plate 15 of the patient table 14.

In step S1, the processor 202 acquires a detection result of the motion sensor. For example, the camera image is acquired from the camera 140.

In step S2, the processor 202 identifies the first motion performed by the subject 130 based on the detection result of the motion sensor acquired in step S1.

In step S3, the processor 202 compares the first motion identified in step S2 with each of the plurality of second motions registered in the database DB stored in the memory 204 or the storage 206.

In step S4, the processor 202 determines whether or not the first motion identified in step S2 matches any of the plurality of second motions registered in the database DB.

In a case where it is determined in step S4 that the first motion matches any of the plurality of second motions stored in the database DB, the processor 202 proceeds to the processing of step S5. In step S5, the processor 202 acquires the notification content corresponding to the second motion that matches the first motion from the database DB.

In a case where the database DB is configured as the trained model, the processor 202 may perform the processing of steps S3 to S5 on the database DB. That is, the processor 202 may input the first motion identified in step S2 to the trained model and acquire the notification content corresponding to the first motion from the trained model.

In step S6, the processor 202 performs the notification based on the notification content acquired in step S5. For example, the processor 202 displays the acquired notification content on the display device 214 as a pop-up screen (an example of "pop-up display").

In a case where it is determined in step S4 that the motion of the subject 130 does not match the plurality of motions stored in the database, and in a case where the notification is performed in step S6, the processor 202 proceeds to the processing of step S7. In step S7, the processor 202 determines whether or not the MRI examination is ended.

In a case where it is determined in step S7 that the MRI examination is ended, the processor 202 ends the processing of the present flowchart. On the other hand, in a case where it is determined in step S7 that the MRI examination is not ended, the processor 202 returns to step S1 and repeats the same processing.

For example, in a case where the subject 130 feels that it is difficult to continue the examination due to deterioration in physical condition, the subject 130 performs the motion of raising the right hand. In step S1, the camera image of the subject 130 who raises the right hand is acquired. In step S2, the first motion performed by the subject 130 is identified as the motion of raising the right hand. In step S3, the motion of raising the right hand identified in step S2 is compared with each of the plurality of second motions registered in the database DB. In step S4, it is determined that the motion of raising the right hand matches the motion of raising the right hand registered in the database DB. In step S5, the notification content corresponding to the motion of raising the right hand is acquired from the database DB. In step S6, the notification is performed based on the notification content acquired in step S5.

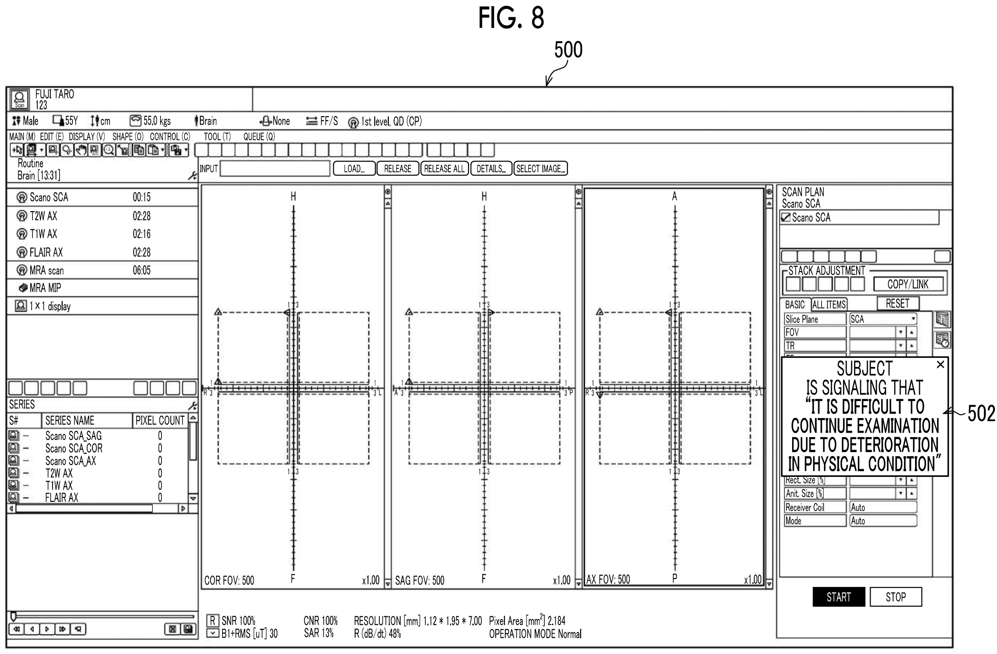

FIG. 8 is a diagram showing an example of a display of a display device 214. In the example shown in FIG. 8, an examination window screen 500 in the MRI examination is displayed on the display device 214. In addition, a pop-up screen 502 smaller than the examination window screen 500 is displayed on the display device 214 in a state of being superimposed on the examination window screen 500. The pop-up screen 502 is a screen for the notification of step S6, and in the example shown in FIG. 8, "The subject is signaling that it is difficult to continue the examination due to deterioration in physical condition" is displayed.

As described above, the subject 130 can communicate the intention or the emotion of the subject to the user from the subject 130 by performing the motion in accordance with the content that the subject 130 wants to notify to the user.

The notification of step S6 is not limited to the example shown in FIG. 8. For example, various aspects are possible for the position and the size of the pop-up screen 502. For example, the pop-up screen 502 may be displayed at the center of the examination window screen 500, or may have a larger size than the example shown in FIG. 8.

In addition, in the database DB shown in FIG. 5, one notification content corresponds to one motion, but the level of the notification to the operator may be changed depending on the magnitude (amplitude) and the duration of the motion even in the same type of motion.

That is, in the database DB, a plurality of magnitudes and notification contents corresponding to the plurality of magnitudes may be registered for one type of motion. For example, "shaking a head slightly left and right" and "shaking a head greatly left and right" may be registered as the motions, and "notifying that the room is hot but can be endured as information" and "notifying that the room is hot and cannot be endured as a warning" may be registered as the notification contents corresponding to "shaking a head slightly left and right" and "shaking a head greatly left and right", respectively.

In addition, in the database DB, a plurality of durations and notification contents corresponding to the plurality of durations may be registered for one type of motion. For example, "shaking a head left and right for 2 seconds" and "shaking a head left and right for 5 seconds" may be registered as the motions, and "notifying that the room is hot but can be endured as information" and "notifying that the room is hot and cannot be endured as a warning" may be registered as the notification contents corresponding to "shaking a head left and right for 2 seconds" and "shaking a head left and right for 5 seconds", respectively.

### Medical Imaging Support Method: Second Embodiment

The medical imaging support method according to the second embodiment notifies the user of predetermined notification content in accordance with the vital information. Here, an example will be described in which the state of the subject 130 (state A) by the motion of the subject 130 and the vital information (state B) of the subject 130 are combined to comprehensively determine the state of the subject 130 and notify the user.

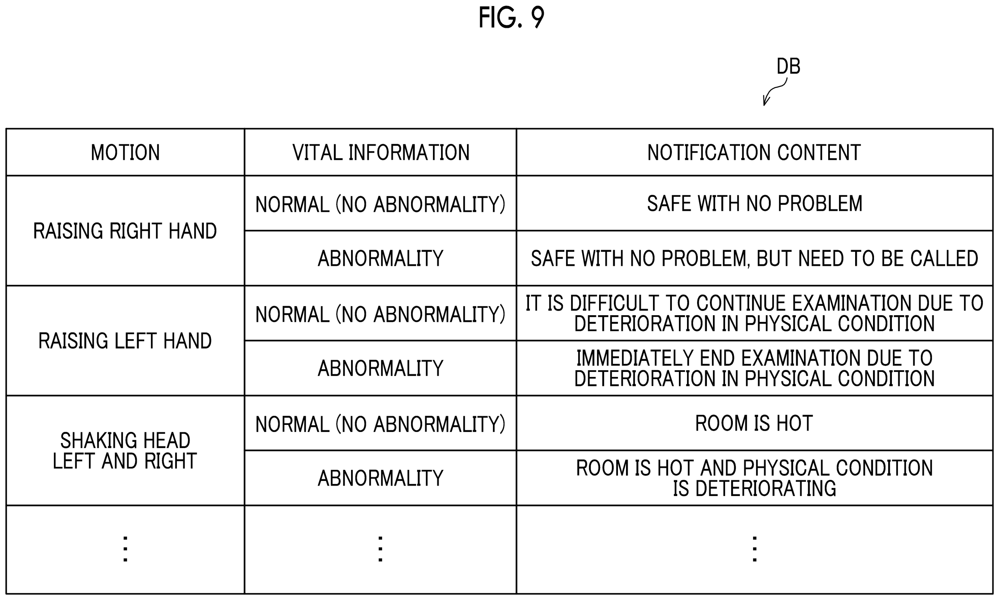

FIG. 9 is a diagram showing an example of registration contents of a database DB used in the medical imaging support method according to the second embodiment. In the database DB, a plurality of motions, vital information, and notification contents to be notified to the user by a combination of the motion and the presence or absence of the abnormality of the vital information are registered in association with each other.

The vital information of the subject 130 includes at least one of a body temperature, a blood pressure, a pulse rate, a heart rate, a respiration rate, the presence or absence of sweating, the number of yawns, or the number of blinks. Here, the vital information is classified into normal (no abnormality) and abnormal. The normal (no abnormality) of the vital information may be a state in which the numerical value of the vital information is within a normal range. The abnormality of the vital information may be a state in which the numerical value is out of the normal range. The vital information may be determined to be abnormal in a case where the vital information of the subject 130 before the examination and the vital information of the subject 130 during the examination are compared and the vital information has changed.

In the database DB shown in FIG. 9, "raising a right hand", "raising a left hand", "shaking a head left and right", ... are registered as the motions, and the normal (no abnormality) and the abnormality of the vital information are combined for each motion, and the corresponding notification content is assigned. For example, in the database DB shown in FIG. 9, in a case where the motion is "raising a right hand" and the vital information is "normal (no abnormality)", "safe with no problem" is registered as the notification content, and in a case where the vital information is "abnormal", "safe with no problem, but need to be called" is registered as the notification content.

In addition, in the database DB shown in FIG. 9, in a case where the motion is "raising a left hand" and the vital information is "normal (no abnormality)", "It is difficult to continue the examination due to deterioration in physical condition" is registered as the notification content, and in a case where the vital information is "abnormal", "immediately end the examination due to deterioration in physical condition" is registered as the notification content.

Further, in the database DB shown in FIG. 9, in a case where the motion is "shaking a head left and right" and the vital information is "normal (no abnormality)", "The room is hot" is registered as the notification content, and in a case where the vital information is "abnormal", "The room is hot and the physical condition is deteriorating" is registered as the notification content.

The pair of the motion and the vital information and the notification content registered in the database DB may be a pair manually registered in advance by the user or the like, or may be a pair of the past motion and the past vital information of the subject and the notification content corresponding to a tendency indicated by the past motion and the past vital information, and may be a pair automatically extracted and registered by the system.

The database DB may be a trained model that has been trained by machine learning. In this case, the trained model is trained by inputting and outputting the pair of the past motion and the past vital information of the subject and the notification content corresponding to the tendency indicated by the past motion and the past vital information. As a result, in a case where the motion and the vital information of the new subject 130 are input, the trained model outputs the notification content.

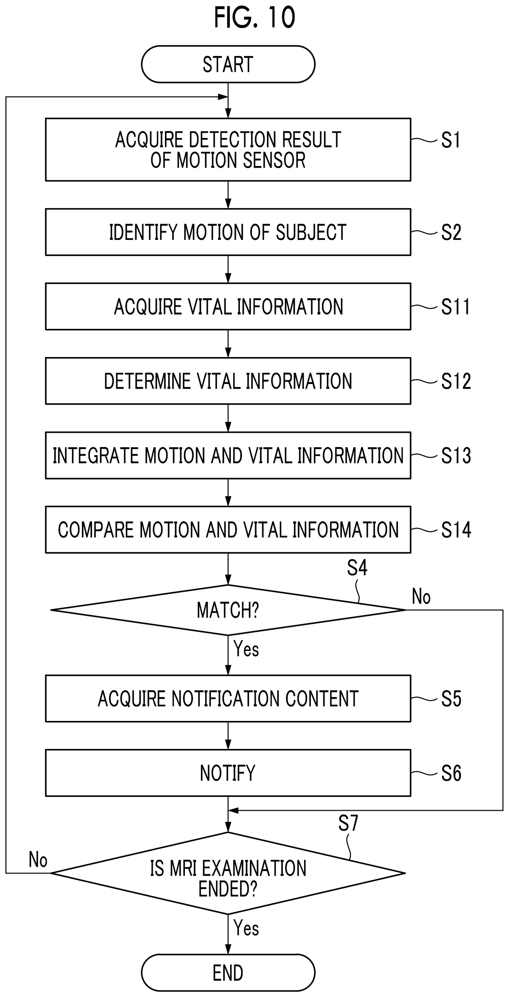

FIG. 10 is a flowchart showing a medical imaging support method according to a second embodiment.

In step S1, the processor 202 acquires a detection result of the motion sensor. In step S2, the processor 202 identifies the first motion performed by the subject 130 based on the detection result of the motion sensor acquired in step S1.

In step S11, the processor 202 acquires the vital information of the subject 130. In step S12, the processor 202 determines the vital information acquired in step S11. Here, the processor 202 determines the presence or absence of the abnormality of the vital information.

In step S13, the processor 202 integrates the first motion identified in step S2 and the vital information determined in step S12. In addition, in step S14, the processor 202 compares the first motion and the vital information integrated in step S13 with each of the plurality of second motions and the vital information registered in the database DB.

In step S4, the processor 202 determines whether or not the first motion and the vital information integrated in step S13 match any of the plurality of second motions and the vital information.

In a case where it is determined in step S4 that the first motion and the vital information match any of the plurality of second motions and the vital information, the processor 202 proceeds to the processing of step S5.

In step S5, the processor 202 acquires the notification content corresponding to the combination of the second motion and the vital information that matches the first motion and the vital information from the database DB. Further, in step S6, the processor 202 performs the notification based on the notification content acquired in step S5.

In a case where it is determined in step S4 that there is no match, and in a case where the notification is performed in step S6, the processor 202 proceeds to the processing of step S7. In step S7, the processor 202 determines whether or not the MRI examination is ended.

In a case where it is determined in step S7 that the MRI examination is ended, the processor 202 ends the processing of the present flowchart. On the other hand, in a case where it is determined in step S7 that the MRI examination is not ended, the processor 202 returns to step S1 and repeats the same processing.

For example, in a case where the user asks the subject 130 a question about the mood using a microphone from the operation room, it is assumed that the subject 130 performs the motion of raising the right hand. This is an expression of intention that state A is "safe with no problem". On the other hand, in a case where the subject 130 also recognizes an abnormality of the vital information such as sweating, blood pressure increase, and an increase in the respiration rate, the subject 130 is actually considered to be "feeling mental pain (tense)" as the state B.

Therefore, in this case, the processor 202 acquires the notification content "safe with no problem, but need to be called" for the combination of the motion "raising a right hand" and the vital information "abnormal" from the database DB shown in FIG. 9, and notifies the user of the content, so that the user can call the subject 130 by saying "I will continue the examination, but you may be a little nervous. Shall we relax by breathing deeply and making it easier to feel?".

### Medical Imaging Support Method: Third Embodiment

In the MRI examination, the imaging site is designated by the user. In the third embodiment, the processor 202 compares the movement of the subject 130 with the imaging site. In a case where the motion of the subject 130 is identified and the part where the movement has occurred is a part other than the imaging site, it is determined that the subject has performed the motion to communicate the intention, and the notification is performed in the same manner as in the first embodiment and the second embodiment.

On the other hand, in a case where the part where the movement has occurred is the imaging site, it is determined that the subject has not performed the motion to communicate the intention, and the user is notified that the subject 130 has a body movement. The notification may be performed by displaying a pop-up screen on the display device 214. As a result, the MRI apparatus 10 can prompt the user to perform re-imaging.

### Functional Configuration of Information Processing Apparatus

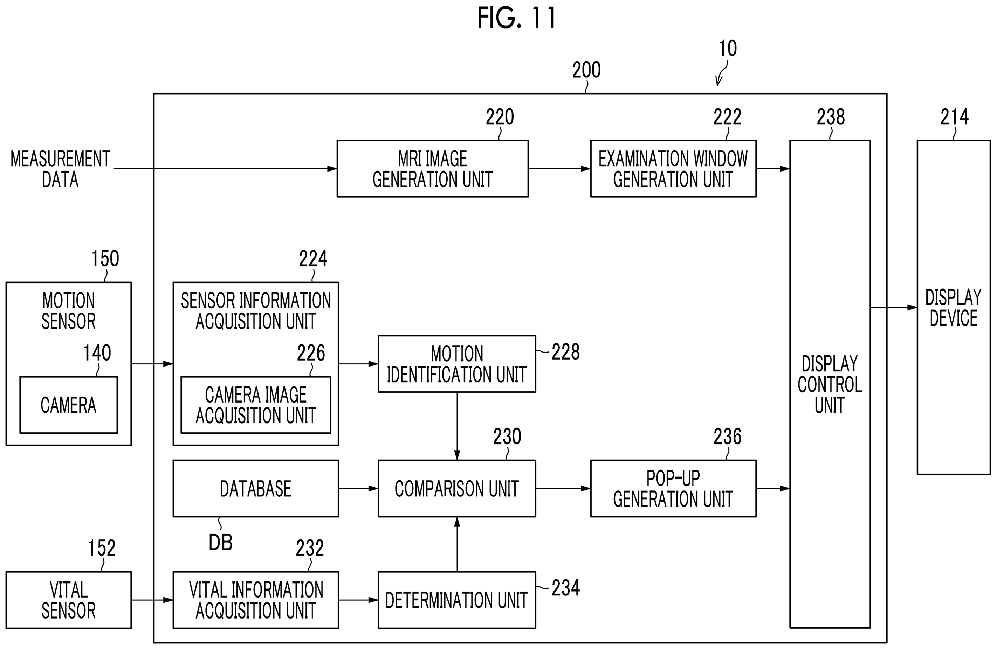

FIG. 11 is a block diagram showing a functional configuration of the information processing apparatus 200 that performs a medical imaging support method. The MRI apparatus 10 includes a motion sensor 150 and a vital sensor 152, which are communicably connected to the information processing apparatus 200, respectively.

The motion sensor 150 detects the motion of the subject 130 and acquires information used to identify the motion of the subject 130. The motion sensor 150 may include any one of the camera 140 that images the subject 130, an embedded sensor disposed on the patient table 14, or a sensor (not shown) attached to the body of the subject 130. The embedded sensor disposed on the patient table 14 is a sensor that can detect a position where the subject 130 and the patient table 14 come into contact or a variation in pressure in a case where the subject 130 moves on the patient table 14, and may be a touch sensor or a pressure sensor. The sensor attached to the body of the subject 130 is a sensor that can detect the movement of the subject 130, and may be at least one of an acceleration sensor, a gyro sensor, a pressure sensor, or a wearable sensor.

The vital sensor 152 detects vital information such as a body temperature, a blood pressure, a pulse rate, a heart rate, a respiration rate, the presence or absence of sweating, the number of yawns, and the number of blinks from the subject 130.

The information processing apparatus 200 includes an MRI image generation unit 220, an examination window generation unit 222, a sensor information acquisition unit 224, a motion identification unit 228, a comparison unit 230, a vital information acquisition unit 232, a determination unit 234, a pop-up generation unit 236, a display control unit 238, and a database DB.

The functions of each processing unit in the information processing apparatus 200 may be implemented by the processor 202 executing the program stored in the memory 204.

The MRI image generation unit 220 performs an image reconstruction process from the measurement data obtained by imaging the subject 130 to generate the MRI image. The examination window generation unit 222 reads the MRI image generated by the MRI image generation unit 220 to generate the examination window screen 500 including the MRI image.

The sensor information acquisition unit 224 acquires the detection result of the motion sensor 150. The sensor information acquisition unit 224 includes a camera image acquisition unit 226. The camera image acquisition unit 226 acquires the camera image captured by the camera 140.

The motion identification unit 228 identifies the motion of the subject 130 based on the sensor information acquired by the sensor information acquisition unit 224. For example, the motion identification unit 228 identifies the motion of the subject 130 from the information acquired from the touch sensor or the pressure sensor.

In addition, the motion identification unit 228 may include an image recognition processing unit that analyzes the camera image acquired by the camera image acquisition unit 226 to identify the motion of the subject 130. For the image recognition technology for identifying the motion of the subject 130 from the camera image, for example, a machine learning algorithm can be applied. That is, the motion identification unit 228 may include a trained model that has been trained through machine learning to identify the motion of the subject 130 from the camera image. The trained model may be, for example, a classification model that is configured by using a convolutional neural network and that performs class classification of the motions. The trained model is not limited to the classification model, and may be, for example, a model that performs an object detection task or a region classification (semantic segmentation) task and may recognize sites such as the head, the abdomen, the arms, and the legs of the subject 130 from the camera image and identify the motion based on a body structure. It should be noted that the trained model is essentially a program.

Additionally, the motion identification unit 228 may calculate an optical flow indicating the movement of the subject 130 between frames based on the camera image and identify the motion of the subject 130 based on a calculation result of the optical flow.

The motion identification unit 228 may combine information acquired by at least one of site specification using a skeleton extraction technique, motion tracking, or pattern recognition by a trained model with the information acquired by the motion sensor 150 to identify the motion of the subject 130.

The comparison unit 230 compares the motion of the subject 130 identified by the motion identification unit 228 with the motion registered in the database DB, determines whether or not the motion of the subject 130 matches the motion registered in the database DB, and acquires the notification content in a case where the motions match. In a case where a plurality of motions are registered in the database DB, the comparison unit 230 extracts the motion that matches the motion of the subject 130 identified by the motion identification unit 228 among the plurality of motions registered in the database DB, and acquires the notification content corresponding to the extracted motion. In a case where the database DB is configured as the trained model, the function of the comparison unit 230 may be included in the database DB.

The vital information acquisition unit 232 acquires the vital information of the subject 130 detected by the vital sensor 152.

The determination unit 234 determines the vital information acquired by the vital information acquisition unit 232. The determination unit 234 determines, for example, the presence or absence of the abnormality of the vital information. The determination result is input to the comparison unit 230.

In a case where the determination result is input from the determination unit 234, the comparison unit 230 integrates the motion of the subject 130 identified by the motion identification unit 228 and the vital information determined by the determination unit 234, compares the integrated motion and vital information with the motion and the vital information registered in the database DB, determines whether or not the motions and the vital information match, and acquires the notification content in a case where the motions and the vital information match. In a case where a plurality of motions and the vital information are registered in the database DB, the comparison unit 230 extracts the motion and the vital information that match the motion of the subject 130 identified by the motion identification unit 228 and the vital information determined by the determination unit 234 among the plurality of motions and the vital information registered in the database DB, and acquires the notification content corresponding to the extracted motion and the vital information.

The pop-up generation unit 236 generates a pop-up screen for notifying the user in accordance with the comparison result of the comparison unit 230. The pop-up generation unit 236 generates a pop-up screen for notifying the user of the notification content acquired by the comparison unit 230.

The display control unit 238 generates data for display on the display device 214. The display control unit 238 displays the examination window screen 500 generated by the examination window generation unit 222 on the display device 214. In addition, the display control unit 238 displays the pop-up screen generated by the pop-up generation unit 236 on the display device 214 in a state of being superimposed on the examination window screen 500.

### Processor

In the present embodiment, each processing is executed by any computer. Additionally, any computer may execute the processing through a processor, a program, or a combination thereof. Any computer may be a general-purpose computer, a computer for a specific application, a system such as a workstation, or other hardware elements capable of executing a program.

The processor may be configured by using one or a plurality of pieces of hardware, and the type of hardware is not limited. For example, the processor may be configured by using hardware such as a central processing unit (CPU), a micro processing unit (MPU), a programmable logic device such as a field programmable gate array (FPGA), a dedicated circuit for executing specific processing such as an application-specific integrated circuit (ASIC), a graphics processing unit (GPU), or a neural processing unit (NPU).

In addition, the processor includes units or means that execute various types of processing in the present embodiment. Further, the type of hardware may be a combination of different types of hardware. In a case where a plurality of pieces of hardware are configured to execute one or a plurality of pieces of processing of a certain processor, the plurality of pieces of hardware may be present in devices physically separated from each other or may be present in the same device. Additionally, in any of the embodiments, the order of each processing by the processor is not limited to the above-described order and may be changed as appropriate. The hardware is configured by using an electrical circuit (circuitry) in which circuit elements such as semiconductor elements are combined.

Further, the present embodiment may be implemented by hardware, software, firmware, microcode, or a combination thereof. Software, firmware, and microcode are configured by programs. In addition, the program may be, for example, a program module group, and each of functions thereof may be implemented by a processor configured to execute each function. The program may be a program code or a plurality of code segments stored in one or a plurality of non-transitory computer-readable media (for example, a storage medium, other storages, or the like). The program may be stored in a plurality of non-transitory computer-readable media present in devices physically separated from each other. The program code or the code segment may represent any combination of procedures, functions, subprograms, routines, subroutines, modules, software packages, classes, or commands, data structures, or program statements. The program code or the code segment may be connected to other code segments or hardware circuits by transmitting and receiving information, data, arguments, parameters, or memory contents.

### Application Example to Program and Program Product

The medical imaging support method according to the embodiment may be configured as a program or a program product for causing a processor or a computer including the processor to implement the functions of each process. The program product is a computer-readable medium that is a tangible and non-transitory information storage medium on which a program is recorded.

A program for causing a computer to implement a part or all of the processing functions of the information processing apparatus 200 can be recorded on a computer-readable medium, which is a non-transitory storage medium such as an optical disk, a magnetic disk, a semiconductor memory, or other tangible media, and the program can be provided through this storage medium.

Additionally, instead of the aspect in which the program is stored in such a tangible and non-transitory computer-readable medium and then provided, program signals can also be provided as a download service using telecommunication lines such as the Internet.

Further, a part or all of the processing functions of the information processing apparatus 200 may be implemented using cloud computing or may be provided as software as a service (SaaS).

### Others

The medical imaging apparatus is not limited to the MRI apparatus 10, and may be various apparatuses such as an X-ray computed tomography (CT) apparatus. The present disclosure is suitable for an apparatus in which imaging is performed in a state in which the subject is not in the visual field of the user, and an apparatus in which a relatively large sound is generated during imaging, making it difficult to communicate the intention by voice.

The technical scope of the present invention is not limited to the scope described in the above-described embodiments. The configuration and the like in each embodiment can be combined between the embodiments as appropriate without departing from the gist of the present invention.

### Explanation of References

10: MRI apparatus
12: gantry
14: patient table
15: top plate
16: leg portion
18: imaging space
102: static magnetic field generating magnet
104: gradient magnetic field coil
106: RF transmit coil
110: receive coil
112: receiver
114: gradient magnetic field power supply
116: high-frequency magnetic field generator
118: sequencer
120: control unit
122: operation unit
130: subject
140: camera
140A: camera
140B: camera
150: motion sensor
152: vital sensor
200: information processing apparatus
202: processor
204: memory
206: storage
208: input/output interface
210: bus
212: input device
214: display device
220: MRI image generation unit
222: examination window generation unit
224: sensor information acquisition unit
226: camera image acquisition unit
228: motion identification unit
230: comparison unit
232: vital information acquisition unit
234: determination unit
236: pop-up generation unit
238: display control unit
500: examination window screen
502: pop-up screen
DB: database
F6A: state of subject
F6B: state of subject
S1 to S7: steps of imaging support method
S11 to S14: steps of imaging support method

## Claims

1. A medical imaging apparatus that captures a medical image of a subject, the medical imaging apparatus comprising:
a gantry having a bore into which the subject placed on a patient table is inserted;
a motion sensor that acquires information used to identify a first motion performed by the subject;
a memory in which a plurality of second motions and notification content corresponding to each of the plurality of second motions are registered; and
a processor,
wherein the processor is configured to:
identify the first motion performed by the subject inserted into the bore based on the information acquired by the motion sensor;
compare the identified first motion with the plurality of second motions registered in the memory; and
notify the user of the notification content corresponding to the second motion that matches the identified first motion among the plurality of second motions registered in the memory.

2. The medical imaging apparatus according to claim 1,
wherein the plurality of second motions include the same type of motions having different magnitudes.

3. The medical imaging apparatus according to claim 1 or 2,
wherein the plurality of second motions include the same type of motions having different durations.

4. The medical imaging apparatus according to any one of claims 1 to 3,
wherein the motion sensor includes any one of an optical camera that images the subject, a sensor disposed on the patient table, or a sensor attached to a body of the subject.

5. The medical imaging apparatus according to claim 4,
wherein the sensor disposed on the patient table includes a touch sensor or a pressure sensor.

6. The medical imaging apparatus according to claim 4,
wherein the sensor attached to the body of the subject includes at least one of an acceleration sensor, a gyro sensor, or a pressure sensor.

7. The medical imaging apparatus according to any one of claims 1 to 6,
wherein the processor is configured to combine information acquired by at least one of site specification using a skeleton extraction technique, motion tracking, or pattern recognition by a trained model with the information acquired by the motion sensor to identify the first motion.

8. The medical imaging apparatus according to any one of claims 1 to 7,
wherein the processor is configured to:
acquire vital information of the subject; and
notify the user of predetermined notification content in accordance with the acquired vital information.

9. The medical imaging apparatus according to any one of claims 1 to 8,
wherein the processor is configured to display the notification content in a pop-up manner on a display device.

10. The medical imaging apparatus according to any one of claims 1 to 9, further comprising:
a static magnetic field generation device that generates a static magnetic field;
a transmission device that irradiates the subject placed in a static magnetic field space with a high-frequency magnetic field pulse; and
a gradient magnetic field generation device that generates a gradient magnetic field in the static magnetic field space.

11. A medical imaging support method of supporting capturing of a medical image of a subject by a medical imaging apparatus including
a gantry having a bore into which the subject placed on a patient table is inserted,
a motion sensor that acquires information used to identify a first motion performed by the subject,
a memory in which a plurality of second motions and notification content corresponding to each of the plurality of second motions are registered, and
a processor, the medical imaging support method comprising:
causing the processor to execute:
identifying the first motion performed by the subject inserted into the bore based on the information acquired by the motion sensor;
comparing the identified first motion with the plurality of second motions registered in the memory; and
notifying the user of the notification content corresponding to the second motion that matches the identified first motion among the plurality of second motions registered in the memory.

12. A non-transitory computer-readable recording medium on which a program for causing a computer to execute the medical imaging support method according to claim 11 is recorded.
